Europäisches Patentamt

European Patent Office

Office européen des brevets

(11) Publication number: **0 300 422 B1**

# EUROPEAN PATENT SPECIFICATION

(45) Date of publication of patent specification: **02.12.92**   (51) Int. Cl.⁵: **C12N 15/34**

(21) Application number: **88111596.8**

(22) Date of filing: **19.07.88**

(54) **An improved method for preparing stable and viable recombinant animal cell viral vectors.**

(30) Priority: **21.07.87 US 77145**

(43) Date of publication of application:
**25.01.89 Bulletin  89/04**

(45) Publication of the grant of the patent:
**02.12.92 Bulletin  92/49**

(84) Designated Contracting States:
**AT BE CH DE ES FR GB GR IT LI LU NL SE**

(56) References cited:

**MOLECULAR AND CELLULAR BIOLOGY, vol. 7, no. 6, June 1987, pages 2087-2096, American Society for Microbiology; B. SAUER: "Functional expression of the cre-lox site-specific recombination system in the yeast Saccharomyces cerevisiae"**

**GENE, vol. 50, December 1986, pages 215-224, Elsevier Science Publishers B.V. (Biomedical Division), Amsterdam, NL; C.L. KEELER, Jr. et al.: "Construction of an infectious pseudorabies virus recombinant expressing a glycoprotein gIII-beta-galactosidase fusion protein"**

**PROC. NATL. ACAD. SCI. USA, vol. 84, no. 24,**

**December 1987, pages 9108-9112; B. SAUER et al.: "Site-specific insertion of DNA into a pseudorabies virus vector"**

(73) Proprietor: **THE DU PONT MERCK PHARMA-CEUTICAL COMPANY**
**Barley Mill Plaza, Building 25**
**Wilmington, Delaware 19880-0025(US)**

(72) Inventor: **Enquist, Lynn William**
**920 Fairthorne Avenue**
**Greenville Delaware 19807(US)**
Inventor: **Robbins, Alan Keith**
**902 North Van Buren Street**
**Wilmington Delaware 19806(US)**
Inventor: **Sauer, Brian Lee**
**4D Presidential Drive**
**Greenville Delaware 19807(US)**
Inventor: **Whealy, Mary Elaine**
**2400 Baynard Boulevard No. 9**
**Wilmington Delaware 19802(US)**

(74) Representative: **von Kreisler, Alek, Dipl.-Chem. et al**
**Patentanwälte Von Kreisler-Selting-Werner, Deichmannhaus am Hauptbahnhof**
**W-5000 Köln 1(DE)**

## Description

Background of the Invention

With the advent of modern molecular biology many attempts have been made to harness viruses for use as vectors for the introduction of foreign DNA into eukaryotic cells, especially animal cells, and for expression of such DNA by those cells. Viruses which have been used in these attempts include SV40, adenovirus, retroviruses, herpes viruses, vaccinia and baculoviruses. Success with these systems has not paralleled that with the much simpler bacterial phages.

A recurring problem with many of these systems is that the introduction of exogenous fragments of DNA is often difficult, inefficient, or both, due to the large size of the vector DNA. For example, the genomes of herpes viruses, typically, contain about 150 kilobases (kb) of DNA. Thus, the traditional in vitro ligation techniques using restriction enzyme/ligase technology are complicated because the viruses have too many restriction enzyme cleavage sites. Introducing heterologous DNA into viral vectors thus has most often relied on homologous recombination which requires that the foreign DNA segment be flanked by DNA homologous to vector DNA. Homologous recombination is inherently inefficient with gene replacement occurring at frequencies of less than about 1 in 100 molecules in the best of systems. Thus, the basic problems have been inability to insert and retrieve any DNA segment with precision, good yields, efficiency and facility. The need remains for a dramatically improved recombination system.

Phage P1 encodes an efficient site-specific recombination system comprising a short asymmetric DNA sequence called loxP and a 38 kilodalton protein called Cre. This bacterial recombination system is described by Sternberg, et al., Bacteriophage P1 site-specific recombination I. Recombination between loxP sites. J. Mol. Biol. 150: 467-486 (1971); Abremski et al., Studies on the Properties of P1 Site-specific Recombination: Evidence for Topologically Unlinked Products Following Recombination. Cell 32: 1301-1311 (1983); Hoess et al., Mechanism of strand cleavage and exchange in the Cre-lox site-specific recombination system. J. Mol. Biol. 181: 351-362 (1985) and Sternberg et al., J. Mol. Biol. 187: 197-212 (1986).

The loxP site is a 34 bp sequence composed of two 13 bp inverted repeats separated by an asymmetric 8 bp core sequence. Recombination between loxP sites in phages 1) can occur either intermolecularly or intramolecularly, 2) can occur when the sites are present on either supercoiled or linear DNA, and 3) is independent of the relative orientation of the loxP sites on the DNA molecule, although such orientation will determine the nature of the recombination. Recombination between two similarly oriented loxP sites on the same DNA molecule results in the deletion of the DNA segment lying between the sites. On a DNA molecule on which the loxP sites are in opposite orientations with respect to each other, recombination results in the inversion of the intervening DNA segment. Bacterial recombination between loxP sites requires only the Cre protein and proceeds efficiently in vitro. There is no requirement for any host factors. A particular advantage of the Cre-lox recombination system is that the Cre protein can be prepared in large quantities from appropriately engineered strains of E. coli as described by Abremski et al., J. Biol. Chem. 259: 1509-1514 (1984).

The Cre-lox system has been extended to effect site-specific recombination of DNA in eukaryotic cells, including yeast and mammalian cells. See commonly assigned U.S. Ser. No. 043,795, filed on April 29, 1987, which is a continuation-in-part of U.S. Ser. No. 784,951, filed on October 7, 1985, both of which are entirely incorporated by reference herein. The vectors used to provide the necessary lox sites and Cre genes are prepared by the conventional methods discussed above, except for one example which employs a specific vector which is part of the invention of this application. Methods of applying Cre-lox to the preparation of stable and viable recombinant animal cell viral vectors have not been suggested prior to this invention.

Summary of the Invention

It is thus an object of this invention to provide a method for site-specific insertion of nucleic acid segments into animal cell viral vectors in a convenient and efficient manner to produce selectable recombinant vectors which are viable and stable.

Upon further study of the specification and appended claims, further objects and advantages of this invention will become apparent to those skilled in the art.

These objects have been achieved by providing a method for preparing a stable and viable recombinant viral vector for animal cells by site-specific insertion of a DNA segment into the DNA of a starting animal cell viral vector, comprising:

contacting Cre with a first lox site which has been introduced into the DNA of said starting animal cell

2

viral vector and with a second lox site in a separate DNA molecule which is circular and which comprises said second lox site adjacent to said DNA segment, whereby Cre effects recombination of said circular DNA with said starting viral vector DNA at said first lox site, and the resultant recombinant animal cell viral vector is viable and stable.

In other aspects, these objects have been achieved by including in this method the additional steps of subsequently selecting for the recombinant vectors, e.g., based on a marker in either the DNA segment or the starting viral vector, preferably the latter, and/or previously inserting a lox site into a viral vector. The invention further relates to the recombinant vectors prepared by the method of the invention, to animal cell viral vectors containing a lox site and to methods of expressing foreign DNA utilizing the recombinant vectors of this invention. A particularly preferred vector is PRV42.

As shown below, it has surprisingly been discovered that the method of this invention proceeds conveniently, rapidly and with a very high efficiency. This significantly simplifies the methodology and shortens the time heretofore necessary to insert foreign DNA into a viral vector, e.g., using the very common restriction endonuclease techniques. For the efficient application of the method of this invention to any animal cell viral vector, it is only necessary to locate in that vector a site for insertion of lox whereupon after recombination the viral vector retains viability. Many such sites in many vectors exist as described below.

This invention thus provides a process for efficiently inserting a fragment of heterologous DNA into a precise location in the DNA of DNA-containing animal viruses wherein a lox fragment of DNA is placed in the DNA of the virus at such a location as not to affect the multiplication of said virus and wherein another lox site is placed adjacent to a fragment of DNA (fragment x) to be inserted into said viral DNA. The latter lox site and said fragment are contained in a circular DNA structure. The lox containing viral DNA is contacted with the circular lox-fragment x DNA in the presence of Cre-protein under suitable known conditions to bring about recombination between the two nucleic acids.

In the preferred embodiment of this invention, a loxC2 site is placed in the gIII gene of the Becker strain of PRV to produce PRV42.

The process of the preferred embodiment of this invention is particularly advantageous for the insertion into PRV viruses of DNA segments which themselves have no selectable markers.

Brief Description of the Drawings

Various other objects, features and attendant advantages of the present invention will be more fully appreciated as the same becomes better understood when considered in conjunction with the accompanying drawings, wherein:

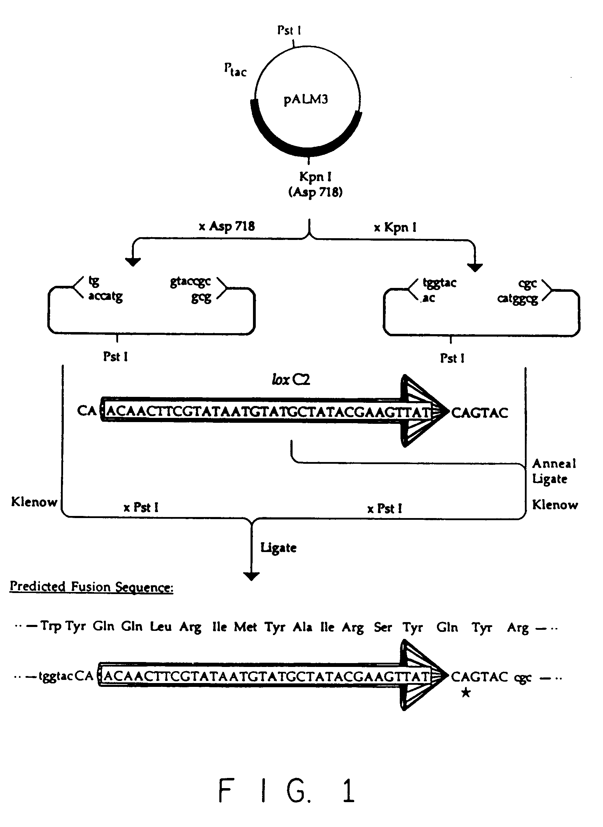

Fig. 1 illustrates construction of the gIII-loxC2 gene. Details of the construction are given in the text. The lox C2 site and its orientation are represented by the large arrow encasing its sequence.

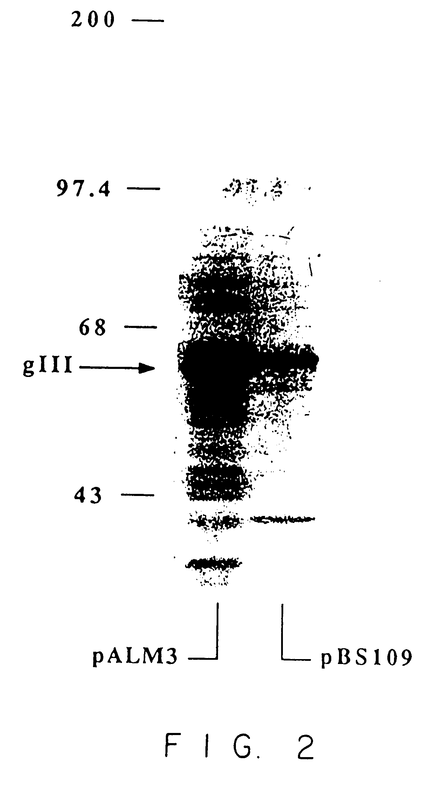

Fig. 2 evidences synthesis of the cro-gIII fusion protein in E. coli. Aggregate protein preparations were electrophoresed on an 8% polyacrylamide gel containing SDS. The arrow indicates the position of the cro-gIII fusion proteins from pALM3 and pBS109. Protein molecular weight markers are displayed to one side.

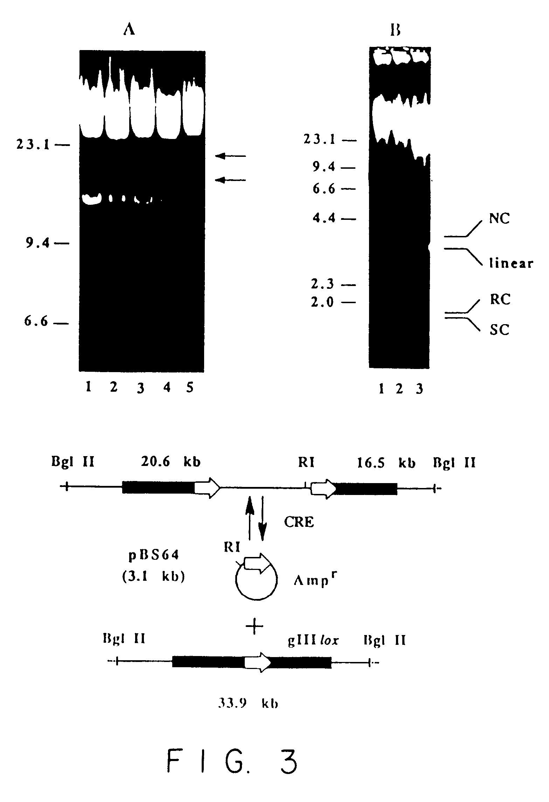

Fig. 3 illustrates Cre mediated recombination in vitro between the circular plasmid pBS64 and the PRV42 genome. The ability of the Cre protein to catalyze the integration of the circular plasmid pBS64 into a linear molecule (PRV42 DNA) containing a lox site in the gIII gene is depicted in the diagram. Similarly, Cre can catalyze excision of a circular molecule from a $lox^2$ substrate, i.e., one which contains 2 directly repeated lox sites.

Panel A shows the integration of pBS64 into PRV42. Cre reactions containing 2 $\mu$g PRV DNA and 0.25 $\mu$g pBS64 were analyzed on a 0.7% agarose gel containing 0.5 $\mu$g/ml ethidium bromide as described by Maniatis et al., Molecular Cloning: a Laboratory Manual (Cold Spring Harbor Laboratory, Cold Spring Harbor, N.Y.) 1982, after digestion with BglII and EcoRI: lane 1, the complete reaction with PRV42; lane 2, PVA omitted; lane 3, Cre omitted; lane 4, pBS64 omitted; lane 5, PRV-Be DNA substituted for PRV42.

Panel B shows the excision of pBS64 from PRV42::pBS64. Cre reactions were performed with 0.6 $\mu$g PRV42::pBS64 DNA and analyzed on a 0.7% agarose gel containing 0.5 $\mu$g/ml ethidium bromide: lane 1, Cre protein omitted: lane 2, the complete reaction; lane 3, as for lane 2 but digested with Bgl II and Eco RI. The mobilities of supercoiled (SC), nicked circular (NC), relaxed circular (RC), and linear (lin) pBS64 are indicated.

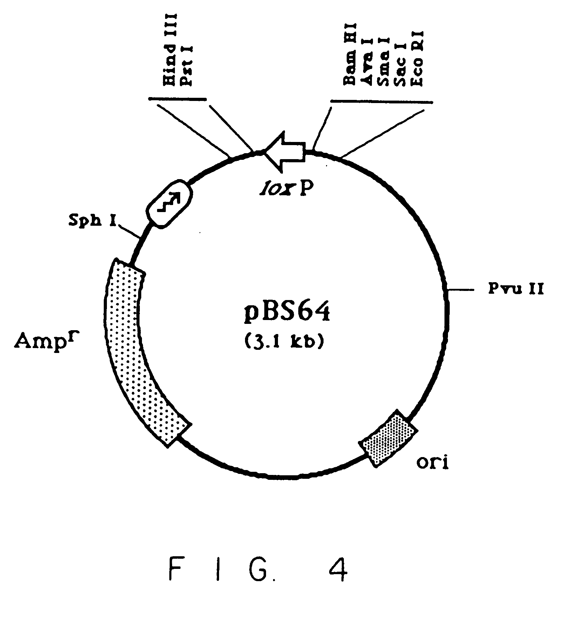

Figure 4 shows a plasmid containing a single loxP site. Plasmid pBS64 is derived from pSP64. It contains a single loxP site inserted in the polylinker region. In addition, the plasmid carries the Amp$^R$

gene and replication origin from pBR322 and an SP6 promoter element (indicated by the open box containing a wavy arrow showing the direction of transcription).

Detailed Description

This invention provides a highly advantageous method of making, selecting and/or using animal viral vectors employing the known Cre-lox technology. It was surprising to find that the inserted DNA segment, flanked by two lox sites remains stably integrated in the resulting virus. Since it is well known that DNA viruses have high rates of recombination (e.g., as shown for Herpes viruses by Ben Porat et al (1985) (see below) and for vaccinia viruses by L.A. Ball, G. of Viral, 61:1788-1795 (1987), it was expected by those skilled in the art that the recombinant vector of this invention would not have been stable and could not have been recovered due to expected excision of it by homologous recombination. This would have been expected to significantly destabilize the system and produce low retrieval rates.

To the contrary, it has now been discovered that the recombinant vectors preparable by this invention are highly stable and viable. As a result, this invention enables site-specific recombination in a viral vector to produce a resultant recombinant animal cell viral vector with high convenience, rapidity and efficiency. This invention enables the insertion and retrieval of essentially any DNA in a specific location (i.e., the lox site) of the virus vector. Efficiencies are typically from 5 to 100-fold superior to those of homologous recombination. Because the recombination events of this invention are so effective, and the product can be relied on to be stable, there is also a resultant significant savings of time in selecting the recombinant product. Thus, this invention not only circumvents the problems discussed above, including those inherent in the use of large DNA viruses as vectors, but does so in a surprisingly advantageous manner.

In accordance with this invention, any known Cre protein, and mutants or variants thereof which retain its functionality with respect to lox sites, can be utilized. Cre proteins are readily available as are lox site DNA segments. The well-known methods of recombinant DNA technology can be utilized in preparing an animal cell viral vector nucleic acid having a lox site and in preparing the separate, circular nucleic acid molecule which is to be inserted therein and which also contains a lox site. See, e.g., Maniatis et al., supra.

One method for preparing the Cre protein is to express it from E. coli. (Abremski et al, supra.) E. coli DH1 and yeast strain BSY90 transformed with plasmid pBS39 (carrying a Cre gene and a GAL1 regulatory nucleotide sequence) have been deposited with the American Type Culture Collection (ATCC) and bear Deposit Accession Numbers ATCC 53255 and ATCC 20772, respectively. Further details on obtaining the Cre protein from these plasmids are given in U.S. Ser. No. 043,795 and are well-known to those of skill in the art.

Details of appropriate lox sites for use in conjunction with this invention are also given in U.S. Ser. No. 043,795. The expression "lox site" includes all nucleotide sequences at which the protein gene product of the Cre gene can catalyze a site-specific recombination. loxP site is a 34 base pair nucleotide sequence which can be isolated from bacteriophage P1 by methods known in the art. One method for isolating a loxP site from bacteriophage P1 is disclosed by Hoess et al., Proc. Natl. Acad.Sci. USA, 79:3398 (1982). The loxP site consists of two base pair inverted repeats separated by an 8 base pair spacer region. The nucleotide sequences of the insert repeats and the spacer region are as follows:

ATAACTTCGTATA ATGTATGC TATACGAAGTTAT

E. coli DH5 Δlac and yeast strain BSY23 transformed with plasmid pBS44 carrying two loxP sites connected with a LEU2 gene have been deposited with the ATCC and bear deposit accession numbers ATCC 53254 and ATCC 20773, respectively. The lox sites can be isolated from plasmid pBS44 with restriction enzymes Eco RI and Sal I, or Xho I and Bam I. In addition, a pre-selected DNA segment can be inserted into pBS44 at either the Sal I or Bam I restriction enzyme sites by techniques known in the art. Other suitable lox sites include loxB, loxL and loxR sites which are nucleotide sequences isolated from E. coli. These sequences are disclosed and described by Hoess et al., Proc. Natl. Acad. Sci USA, 79:3398 (1982).

lox sites can also be produced by a variety of synthetic techniques which are known in the art. Synthetic techniques for producing lox sites are disclosed by Ito et al., Nuc. Acid Res., 10:1755 (1982) and Ogilvie et al., Science, 214:270 (1981). Examples of other such sites are lox Δ86 and lox Δ117. The lox Δ 86 site differs from loxP by having different base pairs at 7 of the 34 base pair positions. Even so, lox Δ86 sites proficiently recombine with loxP sites in Cre-mediated bacterial recombination.

Therefore a lox site is defined herein as a sequence of DNA at which the Cre protein causes site specific DNA recombination either with a site identical to it or with another lox site, e.g., loxP. Moreover, as mentioned, there exist mutants of the cre gene which produce a mutant Cre protein which is still able to cause recombination between lox sites. These are also useful in the invention. The Cre protein is therefore defined as the product of the cre gene (see Sternberg, et al., J. Mol. Biol. 187:197-212 (1986)) or of a mutant cre gene whose product effects site specific recombination at a lox site.

The relatively high efficiency of generating recombinant molecules is an inherent property of the Cre-lox system and surprisingly is preserved in this invention. See., e.g., Hoess et al., Nucl. Acids Res. 14: 2287-2300 (1986) and Sternberg et al., in Mechanisms of DNA Replication and Recombination, UCLA Symposia on Molecular and Cellular Biology, 10: N.R. Cozzarelli, Ed. (New York: Alan R.Liss), pp. 671-684 (1983), describing the efficient recombination caused by Cre at specific lox sites other than loxP.

Conditions which are suitable for the in vitro Cre-lox recombination system are well-known and discussed in the references cited herein, e.g., Abremski et al., J. Biol. Chem. 259: 1509-1514 (1984). Briefly, DNAs to be recombined can be mixed and incubated at 30° for 30 minutes in the presence of 50 mM Tris-HCl, pH 7.5, 33 mM NaCl, 10 mM $MgCl_2$ and purified Cre protein. In the case of intermolecular recombination, the complete reaction also includes 1.7% polyvinyl alcohol (PVA) to facilitate the recombination, presumably by an excluded volume effect as described by Tanford, C., Physical Biochemistry of Macromolecules (Wiley, New York) 1961. The reactions can be stopped by heating the samples at 70° for 5 minutes to inactivate Cre. The DNA in the recombination mixture is then used to transform animal cells or analyzed by digestion with the appropriate restriction enzymes. DNA samples below were extracted with 1 volume of a chloroform/isoamyl alcohol (24/1) mixture before electrophoresis.

Any animal viral vector into which a foreign gene, gene segment or synthetic DNA fragment can be engineered can serve as a recipient of the lox site. Such viral vectors include but are not limited to SV40, Pseudorabies virus (PRV), Polyoma virus, an Adenovirus, Adeno-associated virus, a Herpesvirus, Vaccinia virus, Bovine papilloma virus, Epstein-Barr virus, a baculovirus or a Retrovirus. Features of some of the above named viral vectors are summarized by Weymouth et al., in Mammalian Cell Technology, Ed. W. G. Thilly, pp 39-55 (1986), Butterworth Publishers, 80 Montvale Ave., Stoneham, MA 02180, B. Moss, in Genetically Altered Viruses and the Environment, Banbury Report 22: 291-300 (1985), Cold Spring Harbor Laboratory, Box 100, Cold Spring Harbor, NY 11724, P. M. Howley, in Genetically Altered Viruses and the Environment, Banbury Report 22: 301-312 (1985), Cold Spring Harbor Laboratory, Box 100, Cold Spring Harbor, NY 11724, Logan et al., in Genetically Altered Viruses and the Environment, Banbury Report 22: 313-318 (1985), Cold Spring Harbor Laboratory, Box 100, Cold Spring Harbor, NY 11724, Summers et al., in Genetically Altered Viruses and the Environment, Banbury Report 22: 319-328 (1985), Cold Spring Harbor Laboratory, Box 100, Cold Spring Harbor, NY 11724, and Bernstein et al., in Genetic Engineering: Principles and Methods 7: 235-261 (1985), Plenum Press, 233 Spring Street, New York, NY 10013. See also several papers in Vaccines86, 283-310 (1986), Cold Spring Harbor Laboratory, Box 100, Cold Spring Harbor NY 11724.

In order to achieve the advantages of this invention, including the rapid, convenient, efficient transfer of fragments of foreign DNA into animal viral vectors by means of the Cre-lox system, the vector virus must have a lox site in its DNA and yet be able to replicate. With some viruses, the lox site can be placed directly into the DNA comprising the genome of the virus, e.g., by conventional homologous recombination. For any virus, the first step in getting the lox site in the DNA of the virus vectors can be to put the lox site in a fragment of viral DNA which has been cloned into a plasmid. The lox site-containing fragment of viral DNA must then be incorporated back into the DNA comprising the genome of the virus. This can be accomplished routinely and conventionally by transfecting cells with the lox site-containing plasmid DNA and either infecting with intact virus or transfecting with viral DNA. It is best to use a virus which permits the easy detection of recombinants. A means of detecting recombinants is known for all the viral vectors named above. See the references listed above.

For retroviruses, the insertion of the DNA segment, of course, must be accomplished in the cDNA form of the virus' RNA. After DNA recombination, according to this invention, the retrovirus is then reassembled using fully conventional procedures. For all viruses of this invention, reassembly is in accordance with fully conventional procedures. See Maniatis et al., supra.

For many animal viral vectors, sites have been identified where foreign DNA has been inserted without eliminating the ability of the vector to replicate. See the references listed above.

For example, introduction of foreign genes via live virus infection of eucaryotic cells is a known way to produce desired proteins. The construction of hybrid, Vaccinia viruses by genetic engineering to produce live vaccines was described by Smith et al., Proc. Natl. Acad. Sci. USA, 80:7155-7159, (1983). They inserted and expressed the influenza virus hemagglutinin gene in Vaccinia virus, a virus with a large DNA genome and a non-stringent packaging system that enables the introduction of foreign DNA without loss of infectivity. The foreign gene was inserted into the thymidine kinase gene because that gene is unessential for viral replication and simultaneously provided a selective process for recombinant viruses. Cells infected with the engineered Vaccinia virus produced glycosylated hemagglutinins and the virus successfully immunized hamsters against infection with influenza virus. Subsequent work has described the insertion, and expression of several additional foreign genes in vaccinia virus. The virus is infectious for humans and can be grown in the cells of numerous animal and avian species.

Roizman et al., Science 229:1208-1214 (1985) have described strategies to engineer another large DNA virus, herpes simplex virus type 1 (HSV-1), with the aim of providing vaccines or vectors of genes whose products provide protection against infectious disease. Shih et al., Proc. Natl. Acad. Sci. USA, 81:5867-5870, (1984), also describe an engineered HSV-1. It caused infected cells to produce and excrete the Hepatitis B virus surface antigen for 12 hours. The DNA sequence encoding the hepatitis B virus surface antigen was engineered so that it was under the control of an immediate early promoter of the virus. The inserted surface-antigen gene was regulated as a herpes simplex virus immediate early gene.

Holland et al., J. Virol., 46:649-652, (1983), disclosed that glycoprotein C mutants of herpes simplex virus type 1 were able to multiply in tissue culture cells. A method of selection for the recombinant vectors was disclosed also, i.e., a method of using monoclonal antibodies against glycoprotein C to select infectious virus carrying a variety of mutations in the glycoprotein C gene.

Typical animal cells and their viruses useful in this invention include but are not limited to mammalian cells, e.g., from mice, rats, primates, swine, humans, etc., as well as non-mammalian cells, e.g., insect cells.

Essentially any foreign, exogenous, heterologous, or synthetic DNA fragment can be inserted into the chosen animal viral vector at the specific location of its lox site in accordance with this invention. Such DNA will include that encoding structural proteins, enzymes, regulatory molecules, etc., as well as any fragments thereof. The only requirement for insertion of such DNA into the lox-containing viral vector is that the DNA to be inserted be in circular form adjacent to a lox site. Any of the well-known methods for recombining DNA can be used to prepare the circular nucleic acid. In a preferred aspect of this invention, the inserted DNA segment encodes an antigen whereby the corresponding engineered virus of this invention will be useful as a live vaccine.

It is convenient to generate a circular molecule containing the fragment of heterologous DNA and a lox site by cloning the fragment of heterologous DNA into a plasmid such as pBS64 which contains a lox site. Alternatively, if the fragment of heterologous DNA already exists on a circular plasmid, the lox can be cloned into said plasmid. A third strategy is to insert the fragment of heterologous DNA between two directly repeated lox sites on a DNA molecule, e.g., as found in pBS44 (ATCC 53254). Treatment of this DNA in vitro with Cre protein will cause site-specific recombination between the lox sites and thus generate a circular DNA molecule containing the fragment of heterologous DNA and a lox site.

Similarly, routine and well-known methods can be utilized to insert a lox site into the selected animal viral vector. In order to conveniently and efficiently insert fragments of heterologous DNA into DNA and retrovirus viral vectors, said vectors must first contain a lox site. A lox site can either be obtained by cleaving it from a plasmid containing it, such as pRH43 (described by Sternberg et al., in Mechanisms of DNA Replication and Recombination, UCLA Symposia on Molecular and Cellular Biology 10: 671-684 (1983)) and others discussed herein, by use of appropriate restriction enzymes or by synthesizing it. Methods to accomplish both means of obtaining a lox site are well known in the art.

It is preferred that the DNA fragment comprising the lox site be of such a nature and inserted into recipient DNA in such a way that said lox-containing DNA is in frame with the translation code and does not, upon insertion, create an amber, ochre or other stop codon. In the preferred case discussed below, the fragment of DNA comprising the lox site was synthesized to consist of a lox site flanked by two nucleotides on the 5′ end and six nucleotides on the 3′ end (Figure 1) so that the sequence of the fragment was in frame with the recipient viral DNA and to avoid making a nonsense codon upon insertion. Methods to comply with the above requirements are well known in the art. The lox site, enclosed by the large arrow of Figure 1, which shows the directionality of the lox site, differs from the wild type loxP at a single position: the second base of the lox sequence has been changed from a T to a C. Hence, it is called loxC2. This alteration changes a potential TAA ochre termination signal to a glutamine codon. In addition, the 42-mer was designed to contain a 3′ end which would anneal to a Kpn I site (but not reform a Kpn I site) and a 5′ C residue which allows the regeneration of a Kpn I site at the 5′ end of the lox site. The DNA fragment is inserted into the recipient DNA by use of appropriate restriction, DNA synthetic and ligating enzymes. The DNA fragment comprising the lox site will usually be inserted into recipient viral DNA contained in a plasmid for ease of handling and amplification. Plasmids containing viral vector DNA and means for recovering recombinant viruses are known for all of the viruses mentioned herein as well as many others.

Using the method of this invention, there is provided a significantly improved technique for recombining DNA into animal viral vectors, in comparison with the presently conventional restriction enzyme methods. For example, for a typical restriction method requiring 5-6 days for completion, the method of this invention may require only 3-4 days. For a recombination having a typical efficiency of <1% (e.g., 0.1-1%) using homologous recombination, this invention can perform the same recombination with a significantly increased efficiency, e.g., of up to 50%, typically from 5% up to 50%.

A preferred vector for use according to this invention is derived from the pseudorabies virus (PRV). PRV is an alpha herpesvirus which is also an important pathogen of swine as discussed by Ben-Porat et al., in Molecular Biology of Pseudorabies Virus. Roizman, (Ed.), The Herpesviruses, Plenum Press, New York, pp. 105-173 (1985) and Roizman et al., Intervirology 16: 201-217 (1981). The Pseudorabies virus genome is described by Ben-Porat et al., Virology 41: 265-273 (1970) and in Molecular Biology of Pseudorabies Virus. Roizman, (Ed.), The Herpesvirus, Plenum Press, New York, pp. 105-173 (1985). It is a linear double-stranded DNA molecule, 150 kb in length, containing two regions of unique sequences, $U_L$ and $U_S$, and two 15 kb inverted repeat sequences which bracket the 9 kb $U_S$ sequence.

PRV is preferred for several reasons including the following: its viral DNA is infectious; it infects many types of cultured cells; it is not known to be a human pathogen; it readily and rapidly forms plaques (20-24 hours); it grows rapidly in tissue culture to high titer; it has a high frequency of recombination facilitating genetic construction and analysis; and it has only two DNA structural isomers rather than the four exhibited by herpes simplex virus (see Porat et al., in Molecular Biology of Pseudorabies Virus. Roizman, (Ed.), The Herpesviruses, Plenum Press, New York, pp., 105-173 (1985) for a review of these features of PRV).

Moreover, a facile detection system, which uses specific antibodies to determine the expression of the PRV glycoprotein gIII gene in infected cells, is available (Robbins et al., J. Virol. 59: 635-645 (1986)). The gIII gene is not essential for PRV growth in cultured cells even though it encodes a major viral envelope glycoprotein which is found on the surface of infected cells as pointed out by Robbins et al., J. Virol. 58: 339-347 (1986) and, J. Virol. 59: 635-645 (1986). As the envelope glycoprotein is not essential for viral viability, alterations can be made to the gIII gene which prohibit the synthesis of its envelope glycoprotein or destroy the ability of the envelope glycoprotein produced to react with a particular antibody without destroying the virus' ability to form plaques. These properties of the gIII gene are the basis of a useful method for inserting foreign DNA into the PRV genome by homologous recombination between a suitably tailored DNA fragment containing the gIII gene and viral DNA after co-transformation of the two DNA molecules into mammalian cells as described by Robbins et al., J. Virol. 59: 635-645 (1986). Detecting recombinant plaques is greatly facilitated by the ability to screen many individual plaques, nondestructively, for the presence of gIII antigens. However, the prior art system is relatively inefficient, inconvenient and slow.

The invention provides an especially dramatic improvement over this prior art method of inserting foreign DNA into the preferred PRV vector of this invention. The combination of the unique properties of the PRV system and those of this invention produces a highly advantageous recombinant vector system. In addition to the exceptional facility and efficiency of the recombinant step of this invention, use of PRV provides a simple manner for detecting desired recombinants. Three factors are responsible for the simple selection system. Firstly, insertions of DNA into the PRV gIII gene can be readily detected by the black plaque assay. Secondly, PRV DNA is infectious and hence individual recombinant events can be scored quickly. Moreover, the lox site at which Cre-mediated recombination occurs is sufficiently small and the constraints on the actual nucleotide sequence of the site for recombination are sufficiently nonstringent that lox sites can be designed which allow the in-frame translational fusion of a lox site into the coding region of a protein without grossly altering the structure of that protein as mentioned above. Of course, the method of this invention is generally applicable for the efficient insertion of any lox-containing circular DNA molecule into any non-essential animal viral nucleic acid segment that contains a lox site.

The recombinant animal cell viral vectors preparable by this invention are typically selected for after the Cre-contacting step using any of the known marker selection techniques. The conventional marker can be located on the viral vector and/or on the DNA segment. In an especially preferred embodiment of this invention, the marker is located on the viral vector, e.g., as discussed below for the PRV system. An especially important advantage of this invention where the vector itself contains the selection marker, is that it enables the preparation of recombinant vectors containing any DNA segment irrespective of whether the segment itself contains a marker. While black plaque assays or other assays based on monoclonal antibodies are preferred, any of the conventional marker systems and techniques can be utilized in conjunction with this invention.

In the preferred method of this invention, the synthetic DNA fragment comprising a lox site was inserted into the gIII gene of PRV contained in the DNA of pALM3 (IVI-10049) at the Kpn I site (Figure 1). This was done by annealing and ligating the single-stranded 42-mer to pALM 3 which had been cleaved with Kpn I and filling in the resulting 5$^{'}$ overhang with the Klenow fragment of DNA polymerase. Asp 718, which recognizes the same sequence as Kpn I but which leaves a 5$^{'}$ overhang, was also used to cleave pALM 3 and the 5$^{'}$ overhang was also filled in. Both plasmid digests were then digested with Pst I and the appropriate fragments were purified and ligated to give the construct shown at the bottom of Figure 1. To determine that a functional lox site had been inserted into pALM3 at the Kpn I site and to verify its orientation, a recombination assay was performed in vitro, as described by Abremski et al., J. Biol.Chem. 259: 1509-1514 (1984), with purified Cre protein and a loxP containing tester plasmid, pBS64. This confirmed that the site was indeed functional and that the orientation shown in figure 1 is correct. The resulting plasmid is named pBS109.

In this preferred aspect of this invention, transfer of the gIII-loxC2 gene from the plasmid pBS109 to PRV was facilitated by the properties of the wild type gIII gene and its various mutants as briefly mentioned above. The Becker strain of PRV (PRV-Be) contains a wild type gIII gene that is expressed as a major constituent of the viral envelope and that also is expressed on the surface of infected cells. Consequently, PRV-Be makes a black plaque with the M1 antibody in the black plaque assay. The mutant virus PRV2 (ATCC VR2143) contains a 402 bp deletion within the coding sequence of the gIII gene (5′ to, but not including, the Kpn I site). When reacted with the M1 monoclonal antibody, PRV2 makes a white plaque as described by Robbins et al., J. Virol. 59: 635-645 (1986), because the antibody does not react with the mutant gIII protein formed by PRV2 even though the protein is synthesized. Furthermore, a virus carrying a non-terminating insertion of an Eco RI linker at the KpnI site makes a black plaque with the M1 monoclonal antibody. (See commonly assigned U.S. application Serial No. 886,691 of July 18, 1986.) Moreover, PRV containing the gIII-loxC2 gene as a non-terminating insertion at the KpnI site also makes a black plaque. The latter discovery was most surprising and enables use of the black plaque assay to select PRV-GIII-loxC2 vectors into which a DNA segment has been recombined. This assay is extremely fast and reliable and greatly lowers the time required for a recombination procedure.

Maintenance of a black plaque after insertion of the lox site was determined by cotransfecting PRV2 DNA and an NcoI digest of pBS109 into PK15 cells, preparing a plate stock and then performing a black plaque assay. Should the loxC2 insertion not result in the disruption of the M1 epitope, when PRV gIII-loxC2 recombinants arising by homologous recombination with PRV2 would be expected to give a black-plaque phenotype. This is exactly the result obtained. Of about 1000 plaques screened, 5 were black. In a control experiment in which pBS109 DNA was omitted, no black plaques were obtained after screening 1000 plaques. Virus from three of the black plaques so obtained was plaque purified. Viral DNA was prepared and the presence of a lox site was verified in each isolate by Cre-mediated recombination in vitro (as shown below for one such isolate). One isolate, PRV42, was selected to serve as a viral vector in the examples.

In a preferred insertion of heterologous DNA into a viral vector, per this invention, the DNA in a small circular plasmid, pBS64, adjacent to a lox site was inserted into PRV42. The plasmid pBS64 is pictured in Figure 4. Cre mediated recombination between the loxC2 site on the linear PRV42 genome and the loxP site on the small circular plasmid, pBS64, was carried out, in vitro, as described in the examples and resulted in the integration of the circular plasmid into the PRV genome, as diagramed in Figure 3. To recover virus containing the inserted DNA fragment, DNA from the recombination experiments was conventionally transfected into PK15 cells. Both infective centers and progeny virus were screened by the black plaque assay. The results are shown in Table 1.

Although PRV42 contains a gIII-lox fusion gene and makes black plaques, the insertion of the heterologous DNA into the gIII gene, destroys the M1 epitope of the gIII glycoprotein (the gIII gene product) and results in viruses producing white plaques. This result could not have been predicted. It is however, very beneficial since this feature makes it convenient to screen for viruses containing inserted heterologous DNA. Generally, the production of recombinant virus appears to accurately reflect the amount of recombination detected in vitro.

That the white plaque-forming viruses are indeed the expected recombinants was confirmed by determining the genomic structure of four individual isolates. Each isolate was plaque purified and high titer stocks were prepared on PK15 cells. Viral DNA was derived from each virus and was digested with Eco RI and Bgl II. All four isolates produced the diagnostic restriction fragments described above.

If at any time there is reason to check the fidelity of the fragment of DNA inserted into the animal viral vector per this invention, the use of the in vitro Cre-lox recombination system permits its precise excision and recovery in very convenient fashion. Viral DNA is obtained from the viral vector by known methods and contacted with the Cre protein under the conditions already described. The fragment of DNA between the two lox sites will be excised in the form of a circle and is able to replicate in bacteria. It can easily be recovered for analysis.

In a preferred aspect of the method of this invention this was performed as follows. One recombinant PRV vector from above was isolated and named PRV42::pBS64. The anticipated structure of the gIII region of PRV42::pBS64 is shown in Figure 3. Plasmid pBS64 sequences in PRV42::pBS64 are flanked by directly repeated lox sites. This structure predicts that Cre mediated recombination in vitro should precisely excise the plasmid pBS64 from PRV42::pBS64 and that the resulting excised circle from a linear molecule should be relaxed as described by Abremski et al., J. Biol. Chem. 259: 1509-1514 (1984) and Sternberg et al., in Mechanisms of DNA Replication and Recombination, UCLA Symposia on Molecular and Cellular Biology, 10: N. W. Cozzarelli, Ed. (New York: Alan R. Lis), pp 671-684 (1983).

Cre mediated excision of pBS64 from PRV42::pBS64 DNA regenerates not only pBS64 as a relaxed circular plasmid but also generates a linear PRV molecule which is identical to the parental PRV42. In particular, such PRV molecules should give rise to virus which makes a black plaque when reacted with the M1 monoclonal antibody in the black plaque assay. PRV42::pBS64 DNA was therefore treated with Cre in vitro and then transfected into PK15 cells. The resulting virus was assayed for its ability to produce a black plaque (Table 2).

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius.

The entire disclosure of all applications, patents and publications, if any, cited above and below are hereby incorporated by reference.

EXAMPLES

Animal Cells and Viruses

The swine kidney cells (PK15), the Becker strain of PRV (PRV-Be), PRV2 and their culture have been described previously by Robbins et al., J. Virol. 58: 339-347 (1986) and Robbins et al., J. Virol. 59: 635-645 (1986). The recombinant pseudorabies virus PRV42 carrying a functional lox site was produced by cotransfection of PRV2 DNA and Nco I digested pBS109 DNA into PK15 cells, according to methods described previously by Robbins et al., J. Virol. 58:339-347 (1986). PRV DNA was prepared from nucleocapsids as described by Ben-Porat et al., Virology 41: 265-273 (1970). PRV2 is deposited in ATCC and has deposit accession number VR2143.

Bacterial Strains.

E. coli NF1829 is F′ lacI$^q$ lacZ::Tn5/MC1000. DH5 Δlac is a derivative of DH1 as described by Hanahan, Mol. Biol. 166: 557-580 (1983) and was obtained from M. Berman. Other strains used were HB101, described by Boyer et al., J. Mol. Biol. 41: 459 (1969) and JM101, described by Messing et al., Nucl Acids Res. 9: 309 (1981). Other strains of E. coli which contain the lacI$^q$ gene also can be used for propagating plasmids such as pALM3 and pBS109 which express the gIII gene under the control of a promoter which can be repressed by the lacI$^q$ gene.

Bacterial plasmids

All plasmids were constructed and prepared by standard techniques as described by Robbins et al., J. Virol. 59: 635-645 (1986) and by Maniatis et al., Molecular Cloning: a Laboratory Manual (Cold Spring Harbor Laboratory, Cold Spring Harbor, N.Y.) 1982. Plasmid pALM3, which produces a Cro-gIII fusion protein under the control of the tac promoter, has been described by Robbins et al., J. Virol. 59: 635-645 (1986). pALM3 is described in European Patent Application 0,162,738 dated November 27, 1985 (referred to therein as pNT 7/123). As noted in EPA 0,162,738, E. coli strain K12, NF1829 carrying the plasmid pALM3 (pNT7/123) is on deposit with In Vitro International, Inc., Ann Arbor, MI, bearing the accession number IVI-10049. Plasmid pBS64 was constructed by inserting a loxP site into the polylinker region of pSP64, a plasmid described by Melton et al. Nucl. Acids Res. 12: 7035-7056 (1984). This was accomplished by introducing the 59 base pair (bp) XhoI-Bam HI fragment of pBS30 which contains the loxP site, into the large Sal I-Bam HI fragment of pSP64. This results in a 3.1 kb plasmid containing the SP6 promoter, a loxP site and several unique restriction sites: Hind III, Pst I, Bam HI, Sma I, Sac I and Eco RI. This plasmid is diagrammatically shown in Figure 4. In the examples of this invention, plasmid pBS64 acts as the circular DNA containing a lox site and the additional fragment of DNA to be inserted into the recipient viral vector DNA. However, to practice the invention, of course, any circular fragment of DNA containing a lox site and a fragment of heterologous DNA can be used.

The construction of plasmid pBS109 is diagramed in Figure 1. A 42 nucleotide single-stranded oligomer (that shown above the label loxC2), which comprises a lox site, two 5$'$ bases and six 3$'$ bases, was employed to insert a lox site into the gIII gene. As mentioned above, this lox site (the base sequence inside the arrow of the figure) differs from loxP by the substitution of a C for a T at the second position from the left end of the 34 bp loxP sequence as drawn in Figure 1. This altered site is referred to as loxC2. The 3$'$ end of this oligonucleotide (to the right of the arrow) was designed to anneal to a Kpn I generated DNA end, but does not reform a Kpn I site when it does so. Plasmid pALM3 was digested with Kpn I leaving 3$'$ DNA overhangs at the ends. The single-stranded oligomer was annealed to the Kpn I digested pALM3 DNA and then treated with the Klenow fragment of DNA polymerase I to generate blunt ended, double-stranded DNA. The resulting DNA was digested with PstI into two fragments and the larger of the two was recovered, purified and saved for further use. Separately, pALM3 was digested with Asp 718 which recognizes the same DNA sequence as Kpn I (GGTACC), but which leaves a 5$'$ protruding end rather than a 3$'$ protruding end. The Asp 718 digested pALM3 DNA was similarly treated with the Klenow fragment to fill in the ends. The resulting DNA was then cut at the unique Pst I site and the smaller of the two fragments produced was recovered and purified. The larger fragment from the first PstI digest was then ligated to the smaller fragment from the second PstI digest to produce pBS109. Proper joining of the blunt ends predicts the reconstruction of a Kpn I site 5$'$ to the inserted oligomer. This was verified by digestion with Kpn I.

## Analysis of Cro-gIII fusion proteins in E. coli

A Cro-gIII protein is produced in E. coli from either pALM3 or pBS109 as an insoluble aggregate or inclusion body under the control of the tac promoter as described by Robbins et al., J. Virol. 58: 339-347 (1986). Its production is induced with isopropyl-$\beta$-D-thioigalactopyranoside (IPTG) at a concentration of 1 mM and the aggregate protein is purified, as described by Robbins et al., J. Virol. 58: 339-347 (1986). The presence of the fusion protein is determined by analysis by polyacrylamide gel electrophoresis in the presence of sodium dodecyl sulfate (SDS) as described by Laemmli U.K., Nature (London) 227: 680-685 (1970).

## Detection of recombinant virus.

To detect virus which has an altered gIII protein, the black plaque assay is employed as described by Robbins et al., J. Virol. 58: 339-347 (1986), Holland et al., J. Virol. 46: 649-652 (1983) and Smith et al., J. Immunol. Methods 40: 297-305 (1981), after transfection of DNA into PK15 cells as described by Robbins et al., J. Virol. 59: 635-645 (1986) and Graham et al., Virology 52: 456-467 (1973). This technique uses the M1 monoclonal antibody to gIII, described by Hampl et al., J. Virol. 52: 566-574 (1984), and a horseradish peroxidase linked second antibody to detect the M1 antigen. M1 monoclonal antibody was a gift of Dr. Tamar Ben-Porat, Vanderbilt University from whom it is freely available, and is otherwise readily available, e.g., using well known methods, it can routinely be made by one of skill in the art.

## EXAMPLE 1

$\mu$ = micro when preceding g or 1 (i.e. $\mu$g & $\mu$l)

## Construction of pBS109

The 42 nucleotide single-stranded oligomer loxC2 was synthesized by methods well known in the art (see above) and consists of the sequence of nucleotides shown in Figure 1.

The 42 nucleotide sequence was inserted into the gIII gene of PRV as follows. 3.0 $\mu$g pALM3 was cleaved with 20 units of KpnI for 1 hr at 37°. The reaction was heated for 15 min at 68° and then 1 $\mu$g of the oligonucleotide was mixed with 1.5 $\mu$g of the KpnI digested pALM3 and 200 units of T4 ligase in a volume of 30 $\mu$l. The mixture was incubated overnight at 16°, then heated at 68° for 15 min. Deoxynucleotides at a concentration of 100 $\mu$M and 5 units of the Klenow fragment of DNA polymerase I were added to the mixture, along with the appropriate buffer as described by Maniatis et al., Molecular Cloning: a Laboratory Manual (Cold Spring Harbor Laboratory, Cold Spring Harbor, NY) 1982, to make the final volume 70 $\mu$l. The mixture was incubated for 1 h at 16° and the reaction was stopped by the addition of SDS and heating at 68°. The mixture was extracted with phenol, then two volumes of ethanol were added to precipitate the DNA. The resulting precipitate of DNA was collected. The precipitate was dried and resuspended in 10 $\mu$l of TE (10mM tris HCl, pH 8.0, 1mM EDTA). The DNA was then cleaved with 15 units of PstI in a volume of 25 $\mu$l for 1 h to generate two DNA fragments. Then fragments were separated on an 0.8% agarose gel and the larger fragment was recovered from the gel and resuspended in 10 $\mu$l of TE. This fragment of DNA is referred to as "1A Hi".

3.75 $\mu$g of pALM3 was cleaved with Asp718 in a volume of 15 $\mu$l for 2 h and then heated at 68° for 15 min. Deoxynucleotides and 4 units of the Klenow fragment of DNA polymerase I were added, bringing the volume to 25 $\mu$l and the mixture was incubated for 30 min at 16°, then heated for 20 min at 68°. The DNA was then digested with PstI in a volume of 30 $\mu$l for 2 h at 37° to produce two DNA fragments. These were separated on a 0.8% agarose gel and the smaller fragment was purified and resuspended in 10 $\mu$l of TE. This fragment of DNA is referred to as "Lo".

3.0 ul of "Lo" and 6.0 $\mu$l of "1A Hi" were mixed with 200 units of T4 ligase and T4 ligase buffer as described by Maniatis et al., Molecular Cloning: a Laboratory Manual (Cold Spring Harbor Laboratory, Cold Spring Harbor, NY) 1982, in a total volume of 15 $\mu$l and incubated overnight at 16°. E. coli JM101 was transformed with 5 $\mu$l of the ligation mixture to yield approximately 140 transformants. One of these transformants (No. 6) was shown to contain a functional lox site and was renamed pBS109.

The above reactions are shown diagramatically in Figure 1.

To show that pBS109 contains a lox site, pBS109 DNA was mixed with pBS64 DNA (which contains a single loxP site adjacent to a PstI on a 3.1 kb plasmid) and the mixture was digested with PstI. Subsequent incubation of this mixture with Cre yields diagnostic recombinant DNA fragments 2.3 kb and 7.0 kb in size if a functional lox site is located at the KpnI site of pALM3 in the predicted orientation. This is exactly what was observed.

That the inserted loxC2 site did not introduce a translation termination signal was also shown. If such is the case, pBS109 will express a novel fusion protein in E. coli. The addition of the loxC2 site in-frame at the KpnI site of the gIII gene should result in the insertion of several extra amino acids after amino acid 431 of the predicted 479 amino acid protein. As evidence for this prediction, the size of the protein made from pBS109 was determined. Figure 2 shows that the parental pALM3 makes a gIII protein which has an apparent molecular weight of 57 kilodaltons, as had been demonstrated previously by Robbins et al., J. Virol. 59: 635-645 (1986). The protein expressed by pBS109 migrates as if it were slightly larger than the gIII protein.

## Generation of PRV42

18 $\mu$g of DNA from pBS109 was digested for 4.5 h with 15 units of NcoI and 1 $\mu$g of the digested DNA was mixed with 2 $\mu$g of DNA from PRV2 and transfected into PK15 cells as described by Robbins et al., J. Virol 58: 339-347 (1986). A black plaque assay was performed on the resulting virus progeny. Of 1000 plaques screened, 5 were black. Virus from one black plaque, designated PRV42, was isolated. It was propagated and analyzed.

## Cre-Mediated Insertion of DNA into PRV42

In a total reaction volume of 45 $\mu$l containing Cre buffers, 5.0 $\mu$g of either PRV42 or PRV-Be DNA was mixed with 514 ng pBS64 DNA, 1.7% polyvinyl alcohol and about 60 ng Cre protein. After incubation at 30° for 20 min, the mixture was heated at 70° for 10 min. Two $\mu$g from each reaction mixture was transfected into PK15 cells. Half of the reaction mixture was analyzed for the production of recombinants by digesting the mixture with EcoRI and BglII and separating the resulting DNA fragments on a 0.7% agarose gel. A pictorial visualization of this reaction can be seen in the figures occupying the lower half of Figure 3.

## Results:

Both infective centers and progeny virus were screened by the black plaque assay. The results are shown in Table 1.

| Reaction | Infective Centers | | Virus Stock | | Recombinant DNA Virus | Recombination In vitro |
|---|---|---|---|---|---|---|
| | White | Black | White | Black | (%) | (%) |
| PRV-Be Complete | 0 | 7500 | 0 | 11000 | 0 | 0 |
| PRV-42 Complete | 1 | 960 | 116 | 4312 | 2.6 | 8.1 |
| -PVA | 0 | 4200 | 61 | 5039 | 1 | 5.0 |
| -Cre | 0 | 6500 | 0 | 4300 | 0 | 0 |
| -pBS64 | 0 | 7700 | 0 | 5300 | 0 | 0 |

Table 1. Insertion of plasmid DNA into PRV. The amount of DNA recombination in vitro was determined by measuring the amount of the novel 16.5 kb Bgl II-Eco RI fragment in each reaction in comparison to the 13.2 kb Bgl II F fragment pf PRV as shown in Figure 3.

The production of two new DNA fragments 20.6 kb and 16.5 kb in size is indicative of recombination. Such fragments were found as exemplified in Figure 3A. The amount of recombination which had occurred was determined by densitometry (using a Hoefer GS300 scanning densitometer) of a photographic negative of the ethidium bromide stained gel. The amount of the 16.5 kb recombinant fragment produced was compared to the 13.2 kb BglII fragment of PRV which is not affected by Cre mediated recombination events. Components of the reaction mixture were omitted as indicated. The recombination frequency amounted to 8.1 percent and recombinant virus 2.6 percent.

Excision of pBS64 for PRV42::pBS64

Cre reactions were conducted in a manner identical to those described above for the insertion reactions except that polyvinyl alcohol was omitted and the reaction volume was increased to 135 microliters. The concentration of reactants was the same as for the insertion reactions except that 3.1 μg of PRV42::pBS64 DNA was included. The production of a 3.1 kb pBS64 plasmid is indicative of excision. Because of the unique Eco RI site in pBS64, the excised molecule should be linearized after digestion with Eco RI to produce a 3.1 kb linear fragment. That this occurred is shown in Figure 3B. Incubation of PRV42::pBS64 DNA with Cre in vitro results in the production of a new DNA molecule (lane 2) which comigrates with relaxed pBS64 DNA. The production of this new molecule is dependent upon the Cre protein, as omission of Cre prevents its production (lane 1). The identity of this molecule was confirmed by digesting it with Bgl II and Eco RI (lane 3). The mobility of the resulting molecule shifts to that of linear pBS64, as anticipated. One third of the reaction mixture was transformed into PK15 cells and the resulting viral progeny were analyzed by the black plaque assay. The results are shown in Table 2. Black plaque-forming (recombinant) virus was produced by Cre at a frequency of 56% which is nearly identical to the amount of recombination which had occurred in vitro. The recombination event is completely dependent on the Cre protein. In the absence of Cre, PRV42::pBS64 exhibits a stable white plaque phenotype. This demonstrates that the Cre mediated insertion and excision of DNA occurs precisely at the lox sites. The absence of the formation of black plaques by PRV42::pBS64 which had not been treated with Cre suggests that homologous recombination between the directly repeated 34 base pair lox sites (but containing a 1 base pair mismatch) does not readily occur in mammalian cells. Indeed, approximately 100,000 PRV42::pBS64 plaques have been screened and no black ones have been observed.

Another third of the reaction mixture was run on a 0.7% agarose gel in order to detect the excised circle. The last third of the Cre-reacted DNA was digested with Bgl II and Eco RI and then run on a 0.7% agarose gel in order to detect the characteristic 3.1 kb DNA fragment resulting from linearization of pBS64. The 3.1 kb band observed was quantitated in the manner described for the insertion reaction. The results of the quantitation by gel analysis and the corresponding transformation data are also shown in the Table 2. In

addition, 4.5 $\mu$l of the original reaction mixture was transformed into E. coli HB101 (procedure of Mandel and Higa; note: it is important to use a strain of E. coli which is r⁻, that is deficient in host restriction). That colonies of Ampicillin resistant bacteria are obtained is indicative that the molecule excised by Cre in vitro is indeed circular. Moreover, release of circular plasmid DNA from PRV is a useful technique for recovering DNA which has been passaged through mammalian cells. Such recovered plasmids can thus be easily analyzed for any mutational event which may have occurred in mammalian cells.

TABLE 2

| Reaction | Virus Stock | | Recombinant Virus | DNA Recombination In vitro | Transformants E. coli Amp$^R$/100 |
|---|---|---|---|---|---|
| | Black | White | | | |
| ng | | | (%) | (%) | |
| PRV-42::pBS64 | 0 | 1600 | 0 | 0 | 1 |
| PRV-42::pBS64 +Cre | 1794 | 1468 | 56 | 57 | 434 |

Table 2. Cre-mediated excision of plasmid DNA from PRV. The indicated PRV DNA was treated in vitro as described in Figure 3B. PK15 cells were transfected with 1 μg of DNA to prepare a virus stock which was then analyzed by the black plaque assay. The amount of DNA recombination in vitro was determined by measuring the amount of the linear pBS64 excised in comparison to the 13.2 kb Bgl II F fragment of PRV as shown in Figure 3B. E. coli HB101 was transformed with 100 ng of the indicated PRV-42::pBS64 DNA, which corresponds to 1.2 ng of DNA sequences containing pBS64. In a parallel experiment 1 ng of purified pBS64 DNA gave 1200 colonies either in the presence or absence of exogenous PRV DNA.

The preceding examples can be repeated with similar success by substituting the generically or specifically described reactants and/or operating conditions of this invention for those used in the preceding examples.

From the foregoing description, one skilled in the art can easily ascertain the essential characteristics of this invention, and without departing from the spirit and scope thereof, can make various changes and modifications of the invention to adapt it to various usages and conditions.

## Claims

**Claims for the following Contracting States : AT, BE, CH, DE, FR, GB, IT, LI, LU, NL, SE**

1. A method for preparing a stable and viable recombinant viral vector for animal cells by site-specific insertion of a DNA segment into the DNA of a starting animal cell viral vector, comprising:
   contacting Cre with a first lox site which has been introduced into the DNA of said starting animal cell viral vector and with a second lox site in a separate DNA molecule which is circular and which comprises said second lox site adjacent to said DNA segment, whereby Cre effects recombination of said circular DNA with said starting viral vector DNA at said first lox site, and the resultant recombinant animal cell viral vector is viable and stable.

13

**2.** A method of claim 1, wherein the starting viral vector containing said first lox site comprises a marker which can be used to detect the presence of said inserted DNA segment in said recombinant vector.

**3.** A method of claim 1, wherein at least one of said DNA segment and said starting viral vector DNA containing said first lox site, contains a marker effective to identify recombinant vectors,
and said method further comprises selecting for said recombinant vectors using said marker.

**4.** A method of claim 3, wherein said DNA segment lacks said marker.

**5.** A method of claim 1, further comprising prior to said contacting step, introducing said first lox site in a non-essential region of the DNA of said animal cell viral vector, whereby the resultant vector is viable and stable.

**6.** A method of claim 1, wherein said DNA segment is heterologous, synthetic or exogenous DNA.

**7.** A method of claim 1, wherein said DNA segment encodes a structural protein, an enzyme, a regulatory molecule, or a fragment thereof.

**8.** A method of claim 7, wherein each of said lox sites is a lox P or a loxC2 site.

**9.** A method of claim 1, wherein said viral vector is SV40, Pseudorabies virus (PRV), Polyoma virus, an Adenovirus, Adeno-associated virus, a Herpesvirus, Vaccinia virus, Bovine papilloma virus, Epstein-Barr virus, a baculovirvs or a Retrovirus.

**10.** A method of claim 2, wherein said viral vector is pseudorabies virus (PRV) containing said first lox site in its gIII gene.

**11.** A method of claim 10, wherein said first lox site is a loxC2 site.

**12.** A method of claims 10 or 11, further comprising selecting said recombinant animal cell viral vectors by screening for white plaques in a black plague assay.

**13.** A method of claim 12, wherein said DNA segment lacks a marker effective to identify said recombinant vectors.

**14.** A viable, stable animal cell viral vector containing a lox site introduced into its DNA.

**15.** A vector of claim 14, wherein the virus is SV40, Pseudorabies virus (PRV), Polyoma virus, an Adenovirus, Adeno-associated virus, a Herpesvirus, Vaccinia virus, Bovine papilloma virus, Epstein-Barr virus, a baculovirus or a Retrovirus.

**16.** A vector of claim 14, which is PRV containing a lox site in its gIII gene.

**17.** A recombinant animal cell viral vector produced by the method of any one of claims 1 to 13.

**18.** An animal cell transfected with a viral vector of claim 17.

**19.** In a method comprising expressing a foreign DNA segment in animal cells, the improvement wherein said cells are infected with a viral vector of claim 17 containing said DNA segment.

**Claims for the following Contracting States : ES, GR**

**1.** A process for preparing a stable and viable recombinant viral vector for animal cells by site-specific insertion of a DNA segment into the DNA of a starting animal cell viral vector, comprising:
contacting Cre with a first lox site which has been introduced into the DNA of said starting animal cell viral vector and with a second lox site in a separate DNA molecule which is circular and which comprises said second lox site adjacent to said DNA segment, whereby Cre effects recombination of said circular DNA with said starting viral vector DNA at said first lox site, and the resultant recombinant

14

EP 0 300 422 B1

animal cell viral vector is viable and stable.

2. A process of claim 1, wherein the starting viral vector containing said first lox site comprises a marker which can be used to detect the presence of said inserted DNA segment in said recombinant vector.

3. A process of claim 1, wherein at least one of said DNA segment and said starting viral vector DNA containing said first lox site, contains a marker effective to identify recombinant vectors, and said method further comprises selecting for said recombinant vectors using said marker.

4. A process of claim 3, wherein said DNA segment lacks said marker.

5. A process of claim 1, further comprising prior to said contacting step, introducing said first lox site in a non-essential region of the DNA of said animal cell viral vector, whereby the resultant vector is viable and stable.

6. A process of claim 1, wherein said DNA segment is heterologous, synthetic or exogenous DNA.

7. A process of claim 1, wherein said DNA segment encodes a structural protein, an enzyme, a regulatory molecule, or a fragment thereof.

8. A process of claim 7, wherein each of said lox sites is a lox P or a loxC2 site.

9. A process of claim 1, wherein said viral vector is SV40, Pseudorabies virus (PRY), Polyoma virus, an Adenovirvs, Adeno-associated virus, a Herpesvirvs, Vaccinia virus, Bovine papilloma virus, Epstein-Barr virus, a baculovirus or a Retrovirus.

10. A process of claim 2, wherein said viral vector is pseudorabies virus (PRV) containing said first lox site in its gIII gene.

11. A process of claim 10, wherein said first lox site is a loxC2 site.

12. A process of claims 10 or 11, further comprising selecting said recombinant animal cell viral vectors by screening for white plaques in a black plague assay.

13. A process of claim 12, wherein said DNA segment lacks a marker effective to identify said recombinant vectors.

14. A process for transfecting an animal cell with a viral vector produced in accordance with any one of claims 1 to 13.

15. In a process comprising expressing a foreign DNA segment in animal cells, the improvement wherein said cells are infected with a viral vector produced in accordance with any one of claims 1 to 13 containing said DNA segment.

**Patentansprüche**

**Patentansprüche für folgende Vertragsstaaten : AT, BE, CH, DE, FR, GB, IT, LI, LU, NL, SE**

1. Verfahren zur Herstellung eines stabilen und lebensfähigen rekombinanten viralen Vektors für tierische Zellen durch ortsspezifische Insertion eines DNS-Segments in die DNS eines viralen Vektors einer tierischen Ausgangszelle, umfassend
das Zusammenbringen von Cre mit einer ersten lox-Region, welche in die DNS des viralen Vektors einer tierischen Ausgangszelle eingebracht wurde, und mit einer zweiten lox-Region in einem getrennten DNS-Molekül, welches ringförmig ist und welches die zweite lox-Region neben dem DNS-Segment umfaßt, wobei Cre die Rekombination der ringförmigen DNS mit der DNS des viralen Ausgangsvektors an der ersten lox-Region bewirkt und der daraus folgende rekombinante virale Vektor für eine tierische Zelle lebensfähig und stabil ist.

2. Verfahren des Anspruchs 1, bei welchem der virale Ausgangsvektor, welcher die erste lox-Region

15

enthält, einen Marker enthält, welcher dazu verwendet werden kann, die Anwesenheit des insertierten DNS-Segments in dem rekombinanten Vektor nachzuweisen.

3. Verfahren des Anspruchs 1, bei welchem wenigstens eines (eine) DNS-Segment und virale Ausgangsvektor-DNS, welche die erste lox-Region enthält, einen zum Identifizieren rekombinanter Vektoren wirksamen Marker enthält, und das Verfahren weiterhin das Auswählen der rekombinanten Vektoren mittels des Markers umfaßt.

4. Verfahren des Anspruchs 3, bei welchem dem DNS-Segment der Marker fehlt.

5. Verfahren des Anspruchs 1, welches vor dem Schritt des Zusammenbringens weiterhin das Einführen der ersten lox-Region in eine unwesentliche Region der DNS des viralen Vektors für eine tierische Zelle umfaßt, wobei der daraus folgende Vektor lebensfähig und stabil ist.

6. Verfahren des Anspruchs 1, bei welchem das DNS-Segment eine heterologe, synthetische oder exogene DNS ist.

7. Verfahren des Anspruchs 1, bei welchem das DNS-Segment ein Strukturprotein, ein Enzym, ein Regulatormolekül oder ein Fragment derselben kodiert.

8. Verfahren des Anspruchs 7, bei welchem jede der lox-Regionen eine lox P-Region oder eine loxC2-Region ist.

9. Verfahren des Anspruchs 1, bei welchem der virale Vektor SV40, Pseudotollwut-Virus (PRV), Polyoma-Virus, ein Adeno-virus, Adeno-ähnlicher Virus, ein Herpesvirus, Vaccinia-Virus, Rinderpapilloma-Virus, Epstein-Barr-Virus, ein Baculovirus oder ein Retrovirus ist.

10. Verfahren des Anspruchs 2, bei welchem der virale Vektor Pseudotollwut-Virus (PRV) ist, welcher die erste lox-Region in seinem gIII-Gen enthält.

11. Verfahren des Anspruchs 10, bei welchem die erste lox-Region eine loxC2-Region ist.

12. Verfahren der Ansprüche 10 oder 11, welches weiterhin das Auswählen der rekombinanten viralen Vektoren für eine tierische Zelle durch Screening weißer Plaques in einem Beulenpesttest-Test umfaßt.

13. Verfahren des Anspruchs 12, bei welchem dem DNS-Segment ein Marker fehlt, um die rekombinanten Vektoren wirksam zu identifizieren.

14. Lebensfähiger, stabiler, viraler Vektor für eine tierische Zelle, welche eine in seine DNS eingeführte lox-Region enthält.

15. Vektor des Anspruchs 14, wobei der Virus SV40, Pseudotollwut-Virus (PRV), Polyoma-Virus, ein Adenovirus, Adeno-ähnlicher Virus, ein Herpesvirus, Vaccinia-Virus, Rinderpapilloma-Virus, Epstein-Barr-Virus, ein Baculovirus oder ein Retrovirus ist.

16. Vektor des Anspruchs 14, welcher in seinem gIII-Gen eine lox-Region enthaltender PRV ist.

17. Rekombinanter viraler Vektor für eine tierisch Zelle, welcher nach dem Verfahren einer der Ansprüche 1 bis 13 hergestellt wurde.

18. Mit einem viralen Vektor des Anspruchs 17 transfizierte tierische Zelle.

19. Die Verbesserung eines das Exprimieren eines fremden DNS-Segments in tierischen Zellen umfassenden Verfahrens, wobei die Zellen mit einem das DNS-Segment enthaltenden viralen Vektor des Anspruchs 17 transfiziert werden.

**Patentansprüche für folgende Vertragsstaaten : ES, GR**

1. Verfahren zur Herstellung eines stabilen und lebensfähigen rekombinanten viralen Vektors für tierische Zellen durch ortsspezifische Insertion eines DNS-Segments in die DNS eines viralen Vektors einer tierischen Ausgangszelle, umfassend

das Zusammenbringen von Cre mit einer ersten lox-Region, welche in die DNS des viralen Vektors einer tierischen Ausgangzelle eingebracht wurde, und mit einer zweiten lox-Region in einem getrennten DNS-Molekül, welches ringförmig ist und welches die zweite lox-Region neben dem DNS-Segment umfaßt, wobei Cre die Rekombination der ringförmigen DNS mit der DNS des viralen Ausgangsvektors an der ersten lox-Region bewirkt und der daraus folgende rekombinante virale Vektor für eine tierische Zelle lebensfähig und stabil ist.

2. Verfahren des Anspruchs 1, bei welchem der virale Ausgangsvektor, welcher die erste lox-Region enthält, einen Marker enthält, welcher dazu verwendet werden kann, die Anwesenheit des insertierten DNS-Segments in dem rekombinanten Vektor nachzuweisen.

3. Verfahren des Anspruchs 1, bei welchem wenigstens eines (eine) DNS-Segment und virale Ausgangsvektor-DNS, welche die erste lox-Region enthält, einen zum Identifizieren rekombinanter Vektoren wirksamen Marker enthält, und das Verfahren weiterhin das Auswählen (Suchen nach?) der rekombinanten Vektoren mittels des Markers umfaßt.

4. Verfahren des Anspruchs 3, bei welchem dem DNS-Segment der Marker fehlt.

5. Verfahren des Anspruchs 1, welches vor dem Schritt des in eine unwesentliche Region der DNS des viralen Vektors für Zusammenbringens weiterhin das Einführen der ersten lox-Region eine tierische Zelle umfaßt, wobei der daraus folgende Vektor lebensfähig und stabil ist.

6. Verfahren des Anspruchs 1, bei welchem das DNS-Segment eine heterologe, synthetische oder exogene DNS ist.

7. Verfahren des Anspruchs 1, bei welchem das DNS-Segment ein Strukturprotein, ein Enzym, ein Regulatormolekül oder ein Fragment derselben kodiert.

8. Verfahren des Anspruchs 7, bei welchem jede der lox-Regionen eine lox P-Region oder eine loxC2-Region ist.

9. Verfahren des Anspruchs 1, bei welchem der virale Vektor SV40, Pseudotollwut-Virus (PRV), Polyoma-Virus, ein Adeno-virus, Adeno-ähnlicher Virus, ein Herpesvirus, Vaccinia-Virus, Rinderpapillom*-Virus, Epstein-Barr-Virus, ein Baculovirus oder ein Retrovirus ist.

10. Verfahren des Anspruchs 2, bei welchem der virale Vektor Pseudotollwut-Virus (PRV) ist, welcher die erste lox-Region in seinem gIII-Gen enthält.

11. Verfahren des Anspruchs 10, bei welchem die erste lox-Region eine loxC2-Region ist.

12. Verfahren der Ansprüche 10 oder 11, welches weiter das Auswählen der rekombinanten viralen Vektoren für eine tierische Zelle durch Screening weißer Plaques in einem Pest-Test umfaßt.

13. Verfahren des Anspruchs 12, bei welchem dem DNS-Segment ein Marker fehlt, um die rekombinanten Vektoren wirksam zu identifizieren.

14. Verfahren zum Transfizieren einer tierischen Zelle mit einem viralen Vektor, welcher gemäß einem der Ansprüche 1 bis 13 hergestellt wurde.

15. Die Verbesserung eines das Exprimieren eines fremden DNS-Segments in tierischen Zellen umfassenden Verfahrens, wobei die Zellen mit einem das DNS-Segment enthaltenden viralen Vektor, welcher gemäß einem der Ansprüche 1 bis 13 hergestellt wurde transfiziert sind.

**Revendications**
**Revendications pour les Etats contractants suivants : AT, BE, CH, DE, FR, GB, IT, LI, LU, NL, SE**

1. Une méthode de préparation d'un vecteur viral recombinant stable et viable pour des cellules animales par insertion spécifique de site d'un segment d'ADN dans l'ADN d'un vecteur viral de départ cellules animales consistant à:

   mettre la Cre au contact d'un premier site lox qui a été introduit dans l'ADN de ce vecteur viral de cellule animale de départ et avec un second site lox dans une molécule d'ADN séparée qui est circulaire et qui comprend ce second site lox adjacent à ce segment d'ADN, ce grâce à quoi la Cre effectue une recombinaison de cet ADN circulaire avec cet ADN de vecteur viral de départ sur ce premier site lox et, le vecteur viral de cellule animale recombinant qui en résulte est viable et stable.

2. Une méthode selon la revendication 1, caractérisée en ce que le vecteur viral de départ contenant ce premier site lox comprend un marqueur qui peut être utilisé pour détecter la présence de ce segment d'ADN inséré dans ce vecteur recombinant.

3. Une méthode selon la revendication 1, caractérisée en ce qu'au moins l'un de ces fragments d'ADN et cet ADN de vecteur viral de départ contenant ce premier site lox contient un marqueur efficace pour identifier les vecteurs recombinants et cette méthode comprend en outre la sélection de ces vecteurs recombinants en utilisant ce marqueur.

4. Une méthode selon la revendication 3, caractérisée en ce que ce marqueur fait défaut audit segment d'ADN.

5. Une méthode selon la revendication 1, comprenant en outre avant cette étape de mise en contact, une étape d'introduction de ce premier site lox dans une région non essentielle de l'ADN de ce vecteur viral de cellule animale, ce grâce à quoi le vecteur qui en résulte est viable et stable.

6. Une méthode selon la revendication 4, caractérisée en ce que ce segment d'ADN est un ADN hétérologue, synthétique ou hexogène.

7. Une méthode selon la revendication 1, caractérisée en ce que ce segment d'ADN encode une protéine structurelle, un enzyme, une molécule régulatrice ou un de leurs fragments.

8. Une méthode selon la revendication 7, caractérisée chacun de ces sites lox est un site loxP ou un site loxC2.

9. Une méthode selon la revendication 1, caractérisée en ce que ce vecteur viral est le SV40, le virus de Pseudorabies (PRV), le virus de polyome, un adénovirus, un virus adéno-associé, un virus de l'Herpès, un virus de vaccine, un virus de papillome bovin, un virus d'Epstein-Barr, un baculovirus ou un rétrovirus.

10. Une méthode selon la revendication 2, caractérisée en ce que ce vecteur viral est un virus de pseudorabies (PRV) contenant ce premier site lox dans son gène gIII.

11. Une méthode selon la revendication 10, caractérisée en ce que ce premier site lox est un site loxC2.

12. Une méthode selon la revendication 10 ou 11, caractérisée en outre en ce qu'elle comprend la sélection de ces vecteurs viraux de cellules animales recombinants par criblage des plaques blanches dans un essai à la plaque noire.

13. Une méthode selon la revendication 12, caractérisée en ce que ce segment d'ADN ne comprend pas de marqueur capable d'identifier ces vecteurs recombinants.

14. Un vecteur viral de cellule animale stable et viable contenant un site lox introduit dans son ADN.

15. Un vecteur selon la revendication 14, caractérisé en ce que le virus est SV40, un virus de Pseudorabies (PRV), un virus de polyome, un adénovirus, un virus adéno-associé, un virus de l'Herpès, un virus de vaccine, un virus de papillome bovin, un virus d'Epstein-Barr, un baculovirus ou un rétrovirus.

16. Un vecteur selon la revendication 14, qui est du PRV contenant un site lox dans son gène gIII.

**17.** Un vecteur viral de cellule animale recombinant produit par la méthode selon l'une quelconque des revendications 1 à 13.

**18.** Une cellule animale transfectée par un vecteur viral selon la revendication 17.

**19.** Dans une méthode comprenant l'expression d'un segment d'ADN étranger dans les cellules animales, l'amélioration selon laquelle ces cellules sont infectées par un vecteur viral selon la revendication 17 contenant ce segment d'ADN.

**Revendications pour les Etats contractants suivants : ES, GR**

**1.** Un procédé de préparation d'un vecteur viral recombinant stable et viable pour des cellules animales par insertion spécifique de site d'un segment d'ADN dans l'ADN d'un vecteur viral de cellules animales de départ consistant à:

mettre la Cre au contact d'un premier site lox-qui a été introduit dans l'ADN de ce vecteur viral de cellule animale de départ et avec un second site lox dans une molécule d'ADN séparée qui est circulaire et qui comprend ce second site lox adjacent à ce segment d'ADN, ce grâce à quoi la Cre effectue une recombinaison de cet ADN circulaire avec cet ADN de vecteur viral de départ sur ce premier site lox et le vecteur viral de cellule animale recombinant qui en résulte est viable et stable.

**2.** Un procédé selon la revendication 2, caractérisé en ce que le vecteur viral de départ contenant ce premier site lox comprend un marqueur qui peut être utilisé pour détecter la présence de ce segment d'ADN inséré dans ce vecteur recombinant.

**3.** Un procédé selon la revendication 1, caractérisé en ce qu'au moins l'un de ces fragments d'ADN et cet ADN de vecteur viral de départ contenant ce premier site lox contient un marqueur efficace pour identifier les vecteurs recombinants et cette méthode comprend en outre la sélection de ces vecteurs recombinants en utilisant ce marqueur.

**4.** Un procédé selon la revendication 3, caractérisé en ce que ce marqueur fait défaut audit segment d'ADN.

**5.** Un procédé selon la revendication 1, comprenant en outre avant cette étape de mise en contact, une étape d'introduction de ce premier site lox dans une région non essentielle de l'ADN de ce vecteur viral de cellule animale, ce grâce à quoi le vecteur qui en résulte est viable et stable.

**6.** Un procédé selon la revendication 4, caractérisé en ce que ce segment d'ADN est un ADN hétérologue, synthétique ou hexogène.

**7.** Un procédé selon la revendication 1, caractérisé en ce que ce segment d'ADN encode une protéine structurelle, un enzyme, une molécule régulatrice ou un de leurs fragments.

**8.** Un procédé selon la revendication 7, caractérisé en ce que chacun de ces sites lox est un site loxP ou un site loxC2.

**9.** Un procédé selon la revendication 1, caractérisé en ce que ce vecteur viral est le SV40, le virus de Pseudorabies (PRV), le virus de polyome, un adénovirus, un virus adéno-associé, un virus de l'Herpès, un virus de vaccine, un virus de papillome bovin, un virus d'Epstein-Barr, un baculovirus ou un rétrovirus.

**10.** Un procédé selon la revendication 2, caractérisé en ce que ce vecteur viral est un virus de pseudorabies (PRV) contenant ce premier site lox dans son gène gIII.

**11.** Un procédé selon la revendication 10, caractérisé en ce que ce premier site lox est un site loxC2.

**12.** Un procédé selon la revendication 10 ou 11, caractérisé en outre en ce qu'il comprend la sélection de ces vecteurs viraux de cellules animales recombinants par criblage des plaques blanches dans un essai à la plaque noire.

**13.** Un procédé selon la revendication 12, caractérisé en ce que ce segment d'ADN ne comprend pas de marqueur capable d'identifier ces vecteurs recombinants.

**14.** Un procédé de transfection d'une cellule animale par un vecteur viral produit en accord avec l'une quelconque des revendications 1 à 13.

**15.** Dans un procédé comprenant l'expression d'un segment d'ADN étranger dans des cellules animales, l'amélioration selon laquelle ces cellules sont infectées par un vecteur viral produit en accord avec l'une quelconque des revendications 1 à 13 contenant ce segment d'ADN.

Pst I

P<sub>tac</sub>

pALM3

Kpn I
(Asp 718)

x Asp 718                    x Kpn I

tg                gtaccgc          tggtac          cgc
accatg            gcg              .ac             catggcg

Pst I                            Pst I

lox C2

CA  ACAACTTCGTATAATGTATGCTATACGAAGTTAT  CAGTAC

Anneal
Ligate

Klenow          x Pst I              x Pst I          Klenow

Ligate

Predicted Fusion Sequence:

·· — Trp Tyr Gln Gln Leu Arg Ile Met Tyr Ala Ile Arg Ser Tyr Gln Tyr Arg — ··

·· — tggtac CA  ACAACTTCGTATAATGTATGCTATACGAAGTTAT  CAGTAC cgc — ··
                                                            *

F I G. 1

F I G. 2

F I G. 3

Hind III
Pst I

Bam HI
Ava I
Sma I
Sac I
Eco RI

*lox* P

Sph I

Amp$^r$

pBS64
(3.1 kb)

Pvu II

ori

F I G.  4